# EUROPEAN PATENT APPLICATION

(11) **EP 3 498 256 A1**
(43) Date of publication of application: **19.06.2019**
(21) Application number: 18212038.6
(22) Date of filing: 12.12.2018
(51) Int. Cl.: A61K 8/41, A61K 8/44, A61Q 19/02

(54) **COSMETIC FORMULATION FOR THE REMOVAL OF SKIN STAINS**

(30) Priority: 12.12.2017 IT 201700143180
(71) Applicant: Spray Company SRL, 20121 Milano (IT)
(72) Inventor: PERAZZOLO, Maurizio, 20121 MILANO (IT); GIANCOLA, Simone, 20121 MILANO (IT)
(74) Representative: Allaix, Roberto

(57) **Abstract**

The present invention relates to a cosmetic formulation containing at least one basic amino acid and at least one amino alcohol and at least one cosmetically acceptable excipient, and to its use to reduce the intensity of coloration caused by the application of self-tanning products.

## Description

### FIELD OF INVENTION

This invention relates to a cosmetic formulation for the removal of skin stains.

More specifically, this invention relates to a cosmetic formulation capable of removing skin stains caused by self-tanning compositions containing dihydroxyacetone.

### STATE OF THE ART

In contemporary society, particularly in western countries, the majority of the population of light complexion wants to reach and obtain, especially during the summer holiday periods, but also afterwards, a quick, intense and homogeneous tan.

At the same time, there is a widespread awareness that excessive and prolonged exposure to ultraviolet radiation, whether natural due to exposure to the sun or artificial due to the use of sunbeds or tanning lamps, can cause skin damage and increase the risk of skin cancer.

Faced with these opposing requirements, cosmetic products were developed as early as the 1960s that could give the skin a color similar to that of a natural tan and avoid exposure to ultraviolet radiation, the so-called self-tanning products.

There are different types of self-tanning products on the market that can simulate the natural tan generated by sun exposure. These products generally comprise dihydroxyacetone or DHA, from the English DiHydroxyAcetone, a carbohydrate that gives the skin an amber color, and erythrulose, a sugar that, in contact with oxygen, gives a pinkish color to the surface of the skin, without penetrating deeply and causing damage.

A self-tanner tints the skin through a chemical surface reaction different from the natural tanning process. In the case of dihydroxyacetone (DHA), it reacts with the keratin of the skin causing the formation of dark pigments. The intensity of the color varies according to the concentration of the product in DHA.

The self-tanners of the past have been criticized for developing an un-natural color shade, often too yellowish or orange, and an uneven pigmentation.

Recent formulations have overcome the inconvenience of coloring, thanks to refining techniques that have allowed the achievement of a high level of purity of DHA and the introduction of additives capable of improving the color tone, such as, for example, antioxidant products such as caffeic acid.

Despite the optimization of the formulations, the problems related to the uneven distribution of the self-tanners, often due to the unsuitable use by the inexperienced or distracted consumer, continue to be the cause of the most frequent problems, in particular, with the formation of unsightly darker stains or streaks of the surrounding areas.

In fact, recommendations for the correct use of self-tanning products, such as, for example, make a preventive scrub to the skin, moisturize the skin, apply only on clean and dry skin, blend the product on particularly sensitive parts (eyebrows, hair root, elbows, knees and heels), are often disregarded in whole or in part by consumers.

Since the DHA is colorless during application and the appearance of the coloring takes several hours, it is therefore not possible for the consumer to ascertain the correct distribution of the product on the interested parties. The coloring produced by DHA is very difficult to remove through the use of normal washing with soap and water. Removal requires the use of very aggressive skin cleaners or intensive scrubbing, which can cause skin dryness or even redness and/or injury due to excessive rubbing.

As a result of this, the need arose to produce a product that could remove the stains resulting from the use of DHA-based self-tanning products in a satisfactory manner without being too aggressive towards the skin.

In the art, some products have been described that have addressed this problem.

The international patent application WO2004/058215 A2 described a product based on ammonium oleate in combination with a light abrasive agent, such as pumice powder.

The US patent application US 2007/0258917 A1 described a product based on an abrasive metal oxide, such as magnesium oxide, aluminum oxide or combinations thereof.

In both cases, the use of an abrasive agent has addressed the problem from the point of view of mechanical removal of the stained skin and has not completely eliminated the problems related to possible irritation and sensitization resulting from excessive rubbing.

### SUMMARY OF THE INVENTION

The applicant has addressed the problem described above with the objective of obtaining a product capable of reducing excessive staining and/or streaking caused by uneven application of self-tanning products through a discoloration mechanism capable of not damaging the skin surface and cells.

The applicant noted that the mechanism of action by which DHA acts is completely different from the natural tan based on the synthesis of melanin due to sun exposure.

More specifically, the applicant noted that the coloring obtained by DHA is due to the formation of melanoidins, brownish-brown products resulting from the reaction between the keratin present in the skin and the carbonyl groups of DHA.

The applicant has also observed that DHA is able to penetrate between corneocytes with a marked reactivity given by the presence of hydroxyl groups on the carbon adjacent to the carbonyl group involved in the reaction.

Finally, the applicant noted that the factors influencing the development of melanoidins are extremely variable and comprise the hydration and pH of the stratum corneum, the amount of keratin and the availability of free amino groups, and the concentration of DHA.

On the basis of these observations, the applicant surprisingly found, with a happy intuition, that a composition comprising basic amino acids and/or amino alcohols was able to reduce the intensity of the coloration obtained through the reaction of the DHA with the keratin of the skin.

The applicant has therefore made a cosmetic formulation comprising basic amino acids and amino alcohols and at least one cosmetically acceptable excipient able to reduce the excessive coloring of stains and/or streaks caused by an uneven application of self-tanning products without damaging the surface layer of the skin with aggressive products.

Therefore, according to a first aspect, the present invention relates to a cosmetic formulation containing at least one basic amino acid and at least one amino alcohol and at least one cosmetically acceptable excipient.

In a second aspect, the present invention relates to the use of at least one basic amino acid and at least one amino alcohol to reduce the intensity of coloration caused by the application of self-tanning products.

In a third aspect, the present invention relates to a procedure for reducing the intensity of the coloring resulting from the formation of melanoidins, wherein said procedure comprises the following steps:
- provide a layer affected by melanoidin formation,
- provide a formulation that comprises at least one basic amino acid and at least one amino alcohol, and
- to apply said formulation to said layer.

### SHORT DESCRIPTION OF THE FIGURES

Figure 1 shows a photograph of one foot after treatment with a composition comprising DHA as described in example 3.
Figure 2 shows a photograph of the same foot after treatment with the formulation of the present invention as described in example 3.

### DETAILED DESCRIPTION OF THE INVENTION

For the purposes of the present description and the following claims, except where otherwise stated, all numbers expressing quantities, values, percentages and so on are to be interpreted as changed in all cases by the term "about". In addition, all ranges comprise any combination of the maximum and minimum points described and comprise any intermediate ranges, which may or may not have been specifically listed herein.

The present invention relates to a cosmetic formulation comprising at least one basic amino acid and at least one amino alcohol and at least one cosmetically acceptable excipient.

Amino acids in general are a category of organic chemical compounds characterized by the simultaneous presence in their molecule of a carboxylic group (COOH) and an amino group (NH₂) bound to the same carbon atom.

For the purposes of the present invention, the basic amino acids advantageously used comprise in their molecule a second amino group, said second amino group being able to be a primary amino group ((NH₂), secondary, i.e. with a hydrogen replaced by an organic group comprising a chain of carbon atoms, or tertiary, i.e. with both hydrogen replaced by an organic group comprising a chain of carbon atoms.

Therefore, the amino acids advantageously used in the present invention, can be represented by the following general formula (I): 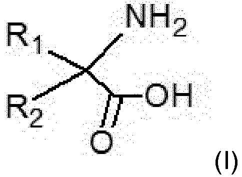 wherein R1 and R2 are hydrogen or any organic group comprising a chain of carbon atoms, linear or branched, saturated or unsaturated, substituted or not substituted, possibly comprising cyclic or heterocyclic, saturated, unsaturated or aromatic groups, and
wherein at least one of R1 and R2 is different from hydrogen and comprises a basic group selected from the group consisting of primary amine group, secondary amine group, and tertiary amine group.

Advantageously, R1 and R2 together comprise a total number of carbon atoms not exceeding 10, preferably not exceeding 8, and more preferably not exceeding 6.

Useful examples of amino acids useful in the present invention are natural amino acids such as arginine, lysine, histidine, and ornithine. Non-natural amino acids comprise homologues such as, for example, 2-amino-3-guanidinopropionic acid, arginine homologue, 2,5-diamino-pentanoic acid, 2,4-diamino-butanoic acid (DAB), and 2,3-diamino-propanoic acid (DAP), homologues of lysine, and derivatives such as, for example, 5-hydroxy-lysine, 4-amino-proline, 3-amino-tyrosine, or N-methyl-histidine.

The cosmetic formulation of this invention comprises an amount of basic amino acid between 0.5 and 15% by weight with respect to the total weight of the formulation.

Preferably, the cosmetic formulation of this invention comprises an amount of basic amino acid between 1 and 10% by weight with respect to the total weight of the formulation.

Advantageously, the cosmetic formulation of this invention comprises an amount of basic amino acid between 1.5 and 5% by weight with respect to the total weight of the formulation.

According to various aspects of the invention, the cosmetic formulation of the present invention comprises an amount of basic amino acid equal to 0.5, 1, 1.5, 2, 2.5, 3, 3.5, 4, 4.5, 5, 5.5, 6, 6.5, 7, 7.5, 8, 8.5, 9, 9.5 and 10% by weight with respect to the total weight of the formulation.

Amino alcohols in general are a category of organic chemical compounds characterized by the simultaneous presence in their molecule of an alcoholic group (OH) and a primary, secondary or tertiary amino group.

Advantageously, the useful amino alcohol in the present invention are represented by the following general formula (II): 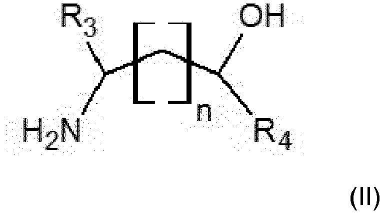 wherein R3 and R4 are hydrogen or any organic group comprising a chain of carbon atoms, linear or branched, saturated or unsaturated, substituted or not substituted, possibly comprising cyclic or heterocyclic, saturated, unsaturated or aromatic groups, and
wherein n is an integer between 0 and 8.

Advantageously, the useful amino alcohols in the present invention comprise a total number of total carbon atoms not exceeding 10, preferably not exceeding 8, and more preferably not exceeding 6. The total number of carbon atoms is advantageously 2, 3, 4 or 5.

Preferably, n is an integer between 0 and 6, more preferably between 0 and 4, and even more preferably between 0 and 2. Advantageously, n can be 0, 1 or 2.

For the purposes of the present invention, the advantageously used amino alcohol comprises 1,2-amino alcohol wherein the amino group and the alcohol group are bound respectively to two adjacent carbon atoms.

Useful examples of amino alcohol useful in this invention are ethanolamines, propanolamines, isopropanolamines, butanolamines, isobutanolamines, tert-butanolamines, pentanolamines, isopentanolamines, 2-methylbutanolamines, 3-methylbutanolamines, and mixtures thereof.

Preferred amino alcohol for the purposes of this invention are ethanolamine, N-methyl-ethanolamine, N,N-dimethylethanolamine, 1-amino-2-propanol, 2-amino-1-propanol, 2-amino-2-methyl-1-propanol, 1-dimethylamino-2-propanol, and mixtures thereof.

The cosmetic formulation of the present invention comprises an amount of amino alcohol between 0.5 and 15% by weight with respect to the total weight of the formulation.

Preferably, the cosmetic formulation of this invention comprises an amount of amino alcohol between 1 and 10% by weight with respect to the total weight of the formulation.

Advantageously, the cosmetic formulation of this invention comprises an amount of amino alcohol between 1.5 and 5% by weight with respect to the total weight of the formulation.

According to various aspects of the invention, the cosmetic formulation of the present invention comprises an amount of amino alcohol equal to 0.5, 1, 1.5, 2, 2.5, 3, 3.5, 4, 4.5, 5, 5.5, 6, 6.5, 7, 7.5, 8, 8.5, 9, 9.5 and 10% by weight with respect to the total weight of the formulation.

According to one aspect of the present invention, the cosmetic formulation of the present invention preferably provides an amount of basic amino acid and amino alcohol such as to provide a ratio by weight between basic amino acid and amino alcohol ranging from 10:1 to 1:10, preferably ranging from 5:1 to 1:5, more preferably ranging from 2:1 to 1:2, and advantageously about 1:1.

The cosmetic formulation of this invention shall also comprise at least one cosmetically acceptable excipient.

In particular, the cosmetic formulation of the present invention may comprise at least one cosmetically acceptable excipient selected from the group consisting of water, emollients, emulsifiers, fillers, dyes, preservatives, and perfumes.

The cosmetic formulation according to the present invention can be in the form of cream, gel, fluid, spray, or soaked wipes.

The cosmetic formulation of this invention is preferably in the form of a water-based emulsion or suspension.

The amount of water in the cosmetic formulation of this invention can vary from 2% to 95%, preferably from 30% to 90%, and more preferably from 40% to 85%, by weight with respect to the total weight of the formulation. Advantageously, the amount of water in the cosmetic formulation of the present invention is comprised from 50% to 75% by weight with respect to the total weight of the formulation. Preferably, filtered and/or distilled water is used, although unfiltered tap water may also be used.

The cosmetic formulation of the present invention has a slightly basic pH, generally equal to or greater than 8, preferably in the range between 8.5 and 11, more preferably between 9 and 10.5, such as for example 9, 9.1, 9.2, 9.3, 9.4, 9.5, 9.6, 9.7, 9.8, 9.9, 10.0, 10.1, 10.2, 10.3, 10.4 and 10.5.

Emollients are generally oily substances that help to smooth and soften the skin, and which can also reduce its roughness, cracks or irritation. Typical suitable emollients comprise mineral oils with viscosity in the range of 50 to 500 centipoise (cps), lanolin oil, coconut oil, cocoa butter, olive oil, almond oil, macadamia nut oil, aloe vera extracts, jojoba oils, safflower oil, corn oil, liquid lanolin, cotton oil and peanut oil.

Solid or semi-solid emollients at room temperature can also be used in sufficient amounts to provide liquid topical compositions. These solid or semi-solid cosmetic emollients comprise hydrogenated lanolin, hydroxylated lanolin, acetylated lanolin, petrolatum, isopropyl lanolate, butyl myristate, cetil myristate, myristil myristate, myristil lactate, cetil alcohol, isostearyl alcohol and isocetil lanolate.

The amount of emollients in the cosmetic formulation of this invention may vary from 1% to 50%, preferably from 2% to 40%, and more preferably from 5% to 30%, by weight with respect to the total weight of the formulation. Advantageously, the amount of emollients in the cosmetic formulation of the present invention is comprised from 10% to 25% by weight with respect to the total weight of the formulation.

The emulsifiers used in the cosmetic formulation of this invention are anionic, cationic, amphoteric and/or non-ionic surfactants. The preferred emulsifiers are non-ionic.

Useful examples of non-ionic surfactants are mono and dialkanolamides of long-chain fatty acids, sorbitan esters, polyglycol esters with fatty acids, ethoxylated fatty alcohols, and acrylic and methacrylic acid polymers comprising ethoxylated fatty alcohols in the side chain.

Particularly suitable are alkyl-polyglycol ethers known by the generic terms "Laureth", "Myristeth", "Oleth", "Ceteth", "Deceth", "Steareth" and "Ceteareth" according to the CTFA nomenclature, followed by a number representing the number of added ethylene oxide molecules, for example "Laureth-16" or "Steareth 20", and their acrylic and methacrylic polymeric derivatives known as "acrylates/steareth-20 itaconate copolymer", "acrylates/ceteth-20 itaconate copolymer", "acrylates/steareth-20 methacrylates copolymer", "acrylates/laureth-25 methacrylates copolymer", "acrylates/beheneth-25 methacrylates copolymer", "acrylates/steareth-20 methacrylates copolymer", and so on. The average degree of ethoxylation varies between about 2.5 and about 25, preferably about 10 and about 20.

The amount of emulsifiers in the cosmetic formulation of this invention may vary from 0.1% to 20%, preferably from 0.5% to 15%, and more preferably from 1% to 30%, by weight with respect to the total weight of the formulation. Advantageously, the amount of emulsifiers in the cosmetic formulation of the present invention is comprised from 1% to 5% by weight with respect to the total weight of the formulation.

Filling or volumizing agents are generally inorganic substances such as clay, talc, mica, perlite, and mixtures thereof.

The term clay comprises mixed silicates of natural or synthetic origin containing different types of cations chosen from alkali metals (e.g. Na, Li, K) or alkaline-earth metals (e.g. Be, Mg, Ca), transition metals and aluminum. Useful clays in the cosmetic formulation of the present invention are kaolin, montmorillonite, beidellite, vermiculite, smectite, sepiolite, or palygorskite, natural or chemically modified, e.g. with acrylic acids, polysaccharides (such as carboxymethylcellulose), and mixtures thereof.

The term talc comprises hydrated magnesium silicates usually consisting of aluminum silicate. The term mica comprises aluminum silicates which optionally comprise iron and/or alkali metals.

Perlite is an expanded material that originates from the heating of volcanic rocks of the obsidian type. It comes in the form of small glass balls with dimensions of the order of 1 - 2 mm.

The amount of fillers or volumizers in the cosmetic formulation of this invention may vary from 0.1% to 20%, preferably from 0.5% to 15%, and more preferably from 1% to 30%, by weight with respect to the total weight of the formulation. Advantageously, the amount of fillers or volumizers in the cosmetic formulation of the present invention is comprised from 1% to 5% by weight with respect to the total weight of the formulation.

Non-exhaustive examples of dyes useful in the formulation of the present invention comprise inorganic and organic pigments.

Inorganic pigments can be white inorganic pigments such as titanium dioxide and zinc oxide; red inorganic pigments such as iron oxide (bengara) and iron titanate; brown inorganic pigments such as iron oxide (maghemite), yellow inorganic pigments such as yellow iron oxide and yellow ochre; black inorganic pigments such as black iron oxide and carbon black; violet inorganic pigments such as mango violet and cobalt violet; inorganic green pigments such as chromium oxide, chromium hydroxide, and cobalt titanate; inorganic blue pigments such as Prussian blue and navy blue; pearl pigments such as mica coated with titanium dioxide, titanium dioxide coated with bismuth oxychloride, bismuth oxychloride, titanium dioxide coated with talcum powder, mica coated with colored titanium dioxide; and other metallic powder pigments such as aluminum powder and copper powder.

Organic pigments may comprise, for example, Red No. 3, Red No. 104, Red No. 106, Red No. 201, Red No. 202, Red No. 204, Red No. 205, Red No. 220, Red No. 226, Red No. 227, Red No. 228, Red No. 230, Red No. 401, Red No. 405, Red No. 505, Orange No. 203, Orange No. 204, Orange No. 205, Yellow No. 4, Yellow No. 5, Yellow No. 202, Yellow No. 203, Yellow No. 205, Yellow No. 401, Blue No. 1, Blue No. 404.

The amount of dyes in the cosmetic formulation of this invention may vary from 0.01% to 10%, preferably from 0.05% to 5%, and more preferably from 0.1% to 3%, by weight with respect to the total weight of the formulation. Advantageously, the amount of colorants in the cosmetic formulation of the present invention is between 0.1% and 1% by weight with respect to the total weight of the formulation.

Non-exhaustive examples of preservatives that may optionally be used in the context of the present invention are quaternary ammonium-based preservatives, such as benzalkonium halides (e.g., benzalkonium chloride ('BAC') and benzalkonium bromide), parabens (e.g. methyl parabens and propyl parabens), phenoxyethanol, benzyl alcohol, chlorobutanol, phenol, sorbic acid, thimerosal, benzoic acid, iodopropinyl butylcarbamate, methylisothiazolinone, methylchloroisothiazolinone or mixtures thereof.

The amount of preservatives in the cosmetic formulation of this invention may vary from 0.01% to 10%, preferably from 0.05% to 5%, and more preferably from 0.1% to 3%, by weight with respect to the total weight of the formulation. Advantageously, the amount of preservatives in the cosmetic formulation of the present invention is between 0.1% and 1% by weight with respect to the total weight of the formulation.

Non-exhaustive examples of fragrances useful in the formulation of this invention are animal fragrances such as musk oil, castoreum, and ambergris, and vegetable fragrances such as nutmeg extract, cardamom extract, ginger extract, cinnamon extract, patchouli oil, geranium oil, orange oil, tangerine oil, orange blossom extract, cedar, tuberose extract, sandalwood oil, bergamot oil, rosemary oil, mint oil, peppermint oil, lemon oil, lavender oil, lemongrass oil, chamomile oil, clove oil, sago oil, neroli oil, cistus oil, eucalyptus oil, mimosa extract, narcissus extract, carrot seed extract, jasmine extract, rose extract and mixture thereof.

The quantity of perfumes in the cosmetic formulation of this invention is generally less than 5%, preferably less than 1%, and more preferably less than 0.1%, by weight with respect to the total weight of the formulation. Advantageously, the amount of preservatives in the cosmetic formulation of the present invention is between 0.1% and 0.01% by weight with respect to the total weight of the formulation.

This invention will be further illustrated hereinbelow by means of a number of preparation examples, which are provided for information purposes only and without any limitation of this invention.

### Example 1 - Formulation example

An example of a cosmetic formulation according to the present invention has been prepared with the components and quantities shown in Table A.

**TABLE A**

| Component | CAS number | Quantity (% w/w) |
|---|---|---|
| Water | 7732-18-5 | 70 |
| Coconut oil | 8001-31-8 | 20 |
| 2-Amino-2-methyl-1-propanol | 124-68-5 | 3 |
| Arginine | 74-79-3 | 3 |
| Acrylates/steareth-20 itaconate copolymer | Not assigned | 2 |
| Limonene | 5989-27-5 | 0.8 |
| Mica | 12001-26-2 | 0.6 |
| Titanium dioxide | 13463-67-7 | 0.5 |
| Orange fragrance | 97766-30-8 | 0.1 |

The product has been subjected to a skin compatibility test and an efficacy test in removing skin stains caused by self-tanning compositions containing dihydroxyacetone, as described in examples 1 and 2 below.

### Example 2 - Skin compatibility assessment

The test was carried out on the product concerned as described in example 1 in order to assess its compatibility with human skin (irritant potential) both under normal conditions of use and under reasonable and foreseeable conditions of misuse.

The product has been used in a single application, in an open-ended epicutaneous test, on the intact skin of the fly area of the forearm of 20 volunteers aged 18 to 65 years of both sexes.

The product was left in contact with the skin for 30 minutes. During and just after 30 minutes of contact with the product, the skin reactivity to the test (usually defined as "open patch test") was evaluated according to the following parameters: erythema, desquamation, edema and vesicles. Each reaction was quantified according to a scale of gravity between zero (no sign of the respective lesion) and 3 (maximum severity of the lesion).

The test was carried out in a single blind mode, under the clinical direction of an allergology specialist with documented experience in clinical trials, and under the supervision of a dermatology specialist.

At the various times of observation (after 5, 10, 15, 20 and 30 minutes from application) the area was carefully examined according to the parameters shown in Table 1. Each parameter was evaluated on a scale from 0 to 3 to express the different levels of severity of the observed reactions.

**TABLE 1**

| Erythema | Edema | Dryness Desquamation | Blisters | Score |
|---|---|---|---|---|
| None | None | No sign | None | 0 |
| Slight erythema, barely visible | Slight oedema (well defined and detected wound edges) | Dryness and slight peeling (smooth skin) | Very small (barely visible) | 1 |
| Modest and uniform reddening | Moderate oedema (lesion edges detected about 1 mm) | Moderate desquamation | Small but clearly visible, with well defined edges | 2 |
| Severe and uniform reddening (with or without interruption and/or scabs) | Severe oedema (lesion edges detected over 1 mm and extended beyond the area exposed to the product) | Severe flaking | Large, well-defined | 3 |

The scores assigned for the parameters considered (erythema, desquamation, edema and blisters) were recorded for each volunteer. The data were considered cumulatively for the entire panel of volunteers and the average was calculated. This has allowed the extrapolation of the Mean Irritation Index (MII), which allows the product to be classified according to the ranges of values indicated in Table 2 below.

**TABLE 2**

| Average Irritation Index - MII | Class |
|---|---|
| ≤ 0.4 | Non-irritant |
| 0.5 ≤ MII ≤ 1.9 | Slightly irritating |
| 2.0 ≤ MII ≤ 4.9 | Modestly irritating |
| 5.0 ≤ MII ≤ 8.0 | Extremely irritating |

The results, summarized in the following table 3, have been interpreted according to the European Standard UNI EN ISO 10993-10 "Biological evaluation of medical devices - Part 10: Tests for irritation and delayed-type hypersensitivity" Edition July 2009 (Chapter 6) which sets the positive threshold at 0.4. Based on this criterion, the effect of modest skin reactivity, which are often observed due to the exaggerated exposure conditions typical of this test, does not affect its final interpretation.

**TABLE 3**

| | Application time | | | | |
|---|---|---|---|---|---|
| | 5 minutes | 10 minutes | 15 minutes | 20 minutes | 30 minutes |
| MII | 0 | 0 | 0.1 | 0.1 | 0.1 |

The results in Table 3 therefore allow the product concerned to be considered as non-irritating.

### Example 3 - Evaluation of effectiveness

The test was carried out on the product concerned as described in example 1 in order to assess its efficacy in removing skin stains caused by self-tanning compositions containing dihydroxyacetone (DHA). The product concerned was used in an in vivo test for the removal of skin stains caused by the application of a preparation containing DHA.

Skin stains were caused using an aqueous solution containing approximately 6.5% by weight of DHA. The solution was applied to the back of the foot of a voluntary subject and then left to work for 24 hours.

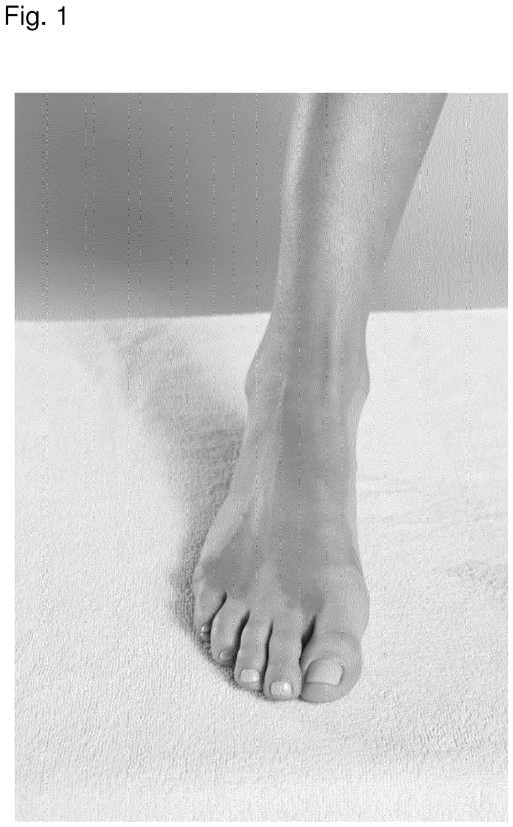

As shown in Figure 1, after 24 hours the stains obtained were evident, and demonstrated the reaction of DHA with the proteins of the keratocytes present in the stratum corneum.

The product concerned, with the formulation of example 1, was applied to the stained areas. The quantity applied was of the order of magnitude of a few grams for an area between 20 and 100 cm². After application with a simple massage, the treated surface was left to rest for 10 minutes to give time to act on the product. Then, the treated surface was cleaned with a soft cloth soaked in lukewarm water, causing a slight rubbing of the skin, and repeating the step several times.

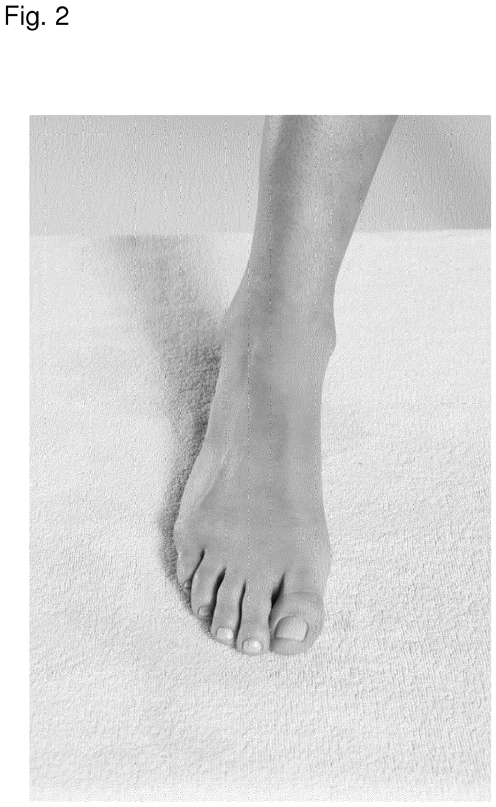

Visual observation already after the first treatment (Figure 2) showed a clear reduction of stains on the skin.

The in vivo test confirmed the tolerability tests shown in example 2, as no adverse skin reactions were detected (such as irritation redness, and so on).

Therefore, in view of the good tolerance of the product, on thicker skin areas (such as the palm of the hand) or for larger and deeper stains, the application and treatment of the product can be repeated until the stains have completely disappeared.

## Claims

1. A cosmetic formulation comprising at least one basic amino acid in an amount of from 0.5 to 15% by weight with respect to the total weight of said formulation and at least one amino alcohol in an amount of from 0.5 to 15% by weight with respect to the total weight of said formulation and at least one cosmetically acceptable excipient.

2. The formulation according to claim 1, wherein said basic amino acid is represented by the following general formula (I): 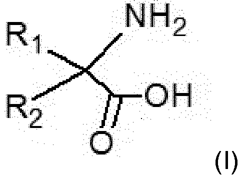 wherein R1 and R2 are hydrogen or any organic group comprising a chain of carbon atoms, linear or branched, saturated or unsaturated, substituted or not substituted, possibly comprising cyclic or heterocyclic, saturated, unsaturated or aromatic groups, and
wherein at least one of R1 and R2 is different from hydrogen and comprises a basic group selected from the group consisting of primary amine group, secondary amine group, and tertiary amine group.

3. The formulation according to claim 2, wherein said R1 and R2 together comprise a total number of carbon atoms not exceeding 10, preferably not exceeding 8, and more preferably not exceeding 6.

4. The formulation according to claim 1, wherein said basic amino acid is selected from the group consisting of natural amino acids and non-natural amino acids, homologues or derivatives.

5. The formulation according to claim 1, wherein said basic amino acid is selected from the group consisting of arginine, lysine, histidine, ornithine, 2-amino-3-guanidinopropionic acid, 2,5-diamino-pentanoic acid, 2,4-diamino-butanoic acid (DAB), 2,3-diamino-propanoic acid (DAP), 5-hydroxy-lysine, 4-amino-proline, 3-amino-tyrosine, and N-methyl-histidine.

6. The formulation according to claim 1, wherein said amino alcohol is represented by the following general formula (II): 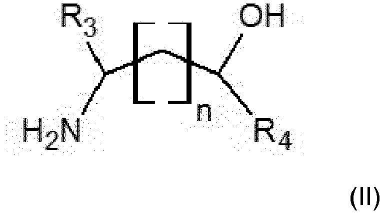 wherein R3 and R4 are hydrogen or any organic group comprising a chain of carbon atoms, linear or branched, saturated or unsaturated, substituted or not substituted, possibly comprising cyclic or heterocyclic, saturated, unsaturated or aromatic groups, and
wherein n is an integer between 0 and 8.

7. The formulation according to claim 6, wherein said amino alcohol comprises a total number of carbon atoms not exceeding 10, preferably not exceeding 8, and more preferably not exceeding 6.

8. The formulation according to claim 6, wherein said n is an integer between 0 and 6, more preferably between 0 and 4, and even more preferably between 0 and 2.

9. The formulation according to claim 1, wherein said amino alcohol is selected from the group consisting of ethanolamines, propanolamines, isopropanolamines, butanolamines, isobutanolamines, tert-butanolamines, pentanolamines, isopentanolamines, 2-methylbutanolamines, 3-methylbutanolamines, and mixtures thereof.

10. The formulation according to claim 1, wherein said amino alcohol is selected from the group consisting of ethanolamine, N-methyl-ethanolamine, N,N-dimethylethanolamine, 1-amino-2-propanol, 2-amino-1-propanol, 2-amino-2-methyl-1-propanol, 1-dimethylamino-2-propanol, and mixtures thereof.

11. The formulation according to claim 1, wherein said cosmetically acceptable excipient is selected from the group consisting of water, emollients, emulsifiers, fillers, dyes, preservatives, and perfumes.

12. The formulation according to claim 1, wherein said formulation is in the form of cream, gel, fluid, spray, or wet wipes.

13. The formulation according to claim 1, wherein said formulation is in the form of a water-based emulsion or suspension.

14. The formulation according to claim 1, wherein said formulation has a slightly basic pH, generally equal to or greater than 8, preferably in the range of from 8.5 to 11, more preferably from 9 to 10.5.

15. The formulation according to claim 1, for use in reducing the intensity of coloration caused by the application of self-tanning products.

16. A procedure for reducing the intensity of coloration resulting from the formation of melanoidins, wherein said procedure comprises the following steps:
• provide a layer affected by melanoidin formation,
• provide a formulation that comprises at least one basic amino acid and at least one amino alcohol as defined in claims 1 to 14, and
• to apply said formulation to said layer.

17. The procedure according to claim 16, wherein said layer consists of the stratum corneum of the skin.
